# EUROPÄISCHE PATENTANMELDUNG

(11) **EP 3 108 898 A1**
(43) Veröffentlichungstag der Anmeldung: **28.12.2016**
(21) Anmeldenummer: 15173274.0
(22) Anmeldetag: 23.06.2015
(51) Int. Cl.: A61K 45/06, A61K 31/045, A61K 31/125, A61K 31/167, A61K 31/192, A61K 31/196, A61K 31/245, A61K 31/405, A61K 31/5415, A61P 19/02, A61P 23/02, A61P 19/04, A61P 21/02, A61P 29/00, A61K 9/08

(54) **PHARMAZEUTISCHE ZUSAMMENSETZUNG**

(71) Anmelder: Michelitsch, Erich, 47447 Moers (DE); Schergens, Thomas, 47829 Krefeld (DE)
(72) Erfinder: Schergens, Thomas, 47829 Krefeld (DE)
(74) Vertreter: DR. STARK & PARTNER

(57) **Zusammenfassung**

Die Erfindung betrifft eine pharmazeutische Zusammensetzung zur äußeren Anwendung, insbesondere zur Behandlung von Schmerzen und/oder Entzündungen, wobei die Zusammensetzung Franzbranntwein und Wirkstoffe umfasst.

Solche Zusammensetzungen werden beispielsweise als Einreibung bei der Therapierung von Zerrungen, Prellungen, Verstauchungen, Muskel- und Gelenkschmerzen eingesetzt. Auch ist ein Einsatz als Prophylaxe möglich, der vorzugsweise bei Gefahr des Wundliegens oder bei mangelhafter Hautdurchblutung erfolgen kann.

Nachteilig hierbei ist, dass die therapeutische Wirkweise eingeschränkt und eher unzureichend ist. Um eine verbesserte pharmazeutische Zusammensetzung anzugeben, sollen die Wirkstoffe zumindest folgende weitere Bestandteile umfassen:
- Kampfer
- Lidocain
- Prilocain und/oder Procain
- eine Schmerzmittelkomponente.

## Beschreibung

Die Erfindung betrifft eine pharmazeutische Zusammensetzung zur äußeren Anwendung, insbesondere zur Behandlung von Schmerzen und/oder Entzündungen, wobei die Zusammensetzung Franzbranntwein und Wirkstoffe umfasst.

Aus der Praxis sind derartige Zusammensetzungen bekannt, die zumeist farblos oder aber grün gefärbt sind. Sie enthalten häufig als Wirkstoffe Wacholderbeeröl, Fichtennadelöl, Latschenkieferöl, Menthol, Kampfer und Thymol, wobei diese teilweise auch als Vergällungsmittel eingesetzt werden.

Solche Zusammensetzungen werden beispielsweise als Einreibung bei der Therapierung von Zerrungen, Prellungen, Verstauchungen, Muskel- und Gelenkschmerzen eingesetzt. Auch ist ein Einsatz als Prophylaxe möglich, der vorzugsweise bei Gefahr des Wundliegens oder bei mangelhafter Hautdurchblutung erfolgen kann.

Nachteilig hierbei ist, dass die therapeutische Wirkweise eingeschränkt und eher unzureichend ist. Aufgabe der Erfindung ist es, die vorgenannten Nachteile zu vermeiden und eine verbesserte pharmazeutische Zusammensetzung anzugeben.

Diese Aufgabe wird bei einer gattungsgemäßen pharmazeutischen Zusammensetzung dadurch gelöst, dass die Wirkstoffe zumindest folgende weitere Bestandteile umfassen:
- Kampfer
- Lidocain
- Prilocain und/oder Procain
- eine Schmerzmittelkomponente.

Anwendungsgebiete für die erfindungsgemäße Zusammensetzung sind Prellungen und Verstauchungen, Muskelfaserrisse sowie Muskelkater, rheumatische Beschwerden sowie chronische oder nicht-chronische Gelenkschmerzen und -entzündungen. Die Anwendung kann bei akuten Beschwerden mehrmals täglich, beispielsweise dreimal täglich, erfolgen, vorzugsweise durch manuelles Auftragen und Verreiben auf die schmerzende Körperstelle bzw. die prophylaktisch zu behandelnde Körperstelle.

Franzbranntwein kann beispielsweise 38-40 Vol.-%, aber auch 70 Vol.-% oder mehr enthalten.

Kampfer wirkt durchblutungsfördernd und lindernd bei chronischer Arthritis und Sehnenscheidenentzündungen, traumatischen Schwellungen, Zerrungen und Verstauchungen und entzündlichen Blutergüssen. In geringer Konzentration hat es eine geringe lokalanästhetische Wirkung und kann eine kühlende Wirkung haben, da es Kälteempfindungen leitende Nervenendigungen reizt. Vorzugsweise kann der Anteil Kampfer, bezogen auf 1 I Franzbranntwein, ca. 15 bis 25 g, vorzugsweise 20 g, betragen.

Lidocain ist ein örtlich wirksames Betäubungsmittel, das bei Auftragung auf die Haut nur an den dort befindlichen Nervenzellen wirkt. Lidocain wirkt dadurch, dass spannungsabhängige Natrium-Kanäle in den Zellmembranen der Nervenzelle blockiert werden, so dass die Erregungsweiterleitung über die Nervenzellen nicht erfolgt, da in die Nervenzelle kein Natrium eintreten kann, wodurch die Entstehung eines Aktionspotentials erschwert wird. Erfindungsgemäß kann der Anteil Lidocain, bezogen auf 1 I Franzbranntwein, ca. 0,5 bis 1,5 g, vorzugsweise 1,0 g, betragen.

Prilocain ist ebenfalls ein Lokalanästhetikum, genauso wie Procain, welches jedoch weniger tief eindringt als Lidocain. Es hat außerdem antientzündliche und parasympatolytische (die Wirkungen eines Anteils des unwillkürlichen Nervensystems hemmende) Eigenschaften und verfügt zudem über perfusionssteigernde Wirkungen, welche sich bei der Neuraltherapie vorteilhaft auswirken.

Dabei kann der Anteil Prilocain und/oder Procain, bezogen auf 1 I Franzbranntwein, ca. 0,1 bis 1,0 g, vorzugsweise 0,2 bis 0,5 g, betragen.

Vorzugsweise kann der Anteil der Schmerzmittelkomponente, bezogen auf 1 I Franzbranntwein, ca. 1,5 bis 2,5 g, vorzugsweise 2,0 g, betragen.

Dabei kann die Schmerzmittelkomponente beispielsweise Diclofenac umfassen. Hierbei handelt es sich um ein Nichtopioid-Analgetikum, welches schmerzauslösende biochemische Prozesse unterdrückt. Diclofenac hat zudem eine fiebersenkende und entzündungshemmende Wirkung und wirkt weiterhin auch antirheumatisch. Der Anteil Diclofenac kann vorteilhafterweise maximal 2,32 Gew.-% betragen.

Stattdessen oder auch ergänzend kann die Schmerzmittelkomponente Ibuprofen umfassen. Ibuprofen ist ein nichtsteroidales Antirheumatikum, das der Behandlung von Schmerzen, Entzündungen und Fieber dient.

Weiterhin kann die Schmerzmittelkomponente, entweder alternativ oder in Kombination mit Diclofenac und/oder Ibuprofen, Naproxen umfassen. Naproxen wirkt schmerzlindernd und entzündungshemmend sowie fiebersenkend.

Erfindungsgemäß kann die Schmerzmittelkomponente Acemetacin umfassen, bei dem es sich ebenfalls um ein Nichtopioid-Analgetikum handelt. Dieses kann alternativ zu den vorgenannten Stoffen oder aber in Kombination mit einem oder mehreren der vorerwähnten anderen Bestandteile in der Schmerzmittelkomponente vorgesehen sein.

Vorteilhafterweise kann die Schmerzmittelkomponente Indometacin umfassen, bei dem es sich ebenfalls um ein nichtsteroidales Antirheumatikum handelt. Dieses kann alternativ zu den vorgenannten Stoffen oder aber in Kombination mit einem oder mehreren der vorerwähnten anderen Bestandteile in der Schmerzmittelkomponente vorgesehen sein.

Erfindungsgemäß kann die Schmerzmittelkomponente Meloxicam umfassen, bei dem es sich ebenfalls um ein nichtsteroidales Antirheumatikum handelt. Dieses kann alternativ zu den vorgenannten Stoffen oder aber in Kombination mit einem oder mehreren der vorerwähnten anderen Bestandteile in der Schmerzmittelkomponente vorgesehen sein.

Bei einem bevorzugten Ausführungsbeispiel der Erfindung kann die Zusammensetzung flüssig, vorzugsweise dünnflüssig, oder gelförmig sein, so dass eine einfache äußerliche Applikation durch Einreiben möglich ist. Sofern die Zusammensetzung gelförmig ist, kann sie Methlyhydroxypropylcellulose enthalten, welches ein Verdickungsmittel und Emulsionsstabilisator ist und zudem auch als Feuchthaltemittel wirkt und gut hautverträglich ist.

Bei einem bevorzugten Ausführungsbeispiel der Erfindung kann die Sprühdose mit einer erfindungsgemäßen flüssigen pharmazeutischen Zusammensetzung befüllt sein, so dass die Zusammensetzung als aus der Sprühdose austretendes Aerosol aufgebracht werden kann. Hierdurch ist ein besonders einfaches Auftragen möglich, wobei sogar schwer erreichbare Körperpartien noch besprüht werden können, und es resultiert automatisch eine sehr gleichmäßige Verteilung.

Nachstehend werden einige beispielhafte Ausprägungen der erfindungsgemäßen pharmazeutischen Zusammensetzung zur äußeren Anwendung angegeben:
Zusammensetzung 1:
   - 1 I Franzbranntwein
   - 20 g Kampfer
   - 2 g Diclofenac
   - 1 g Lidocain
   - 0,2 bis 0,5 g Prilocain
Zusammensetzung 2:
   - 1 I Franzbranntwein
   - 20 g Kampfer
   - max. 400 mg Ibuprofen
   - 1 g Lidocain
   - 0,2 bis 0,5 g Prilocain
Zusammensetzung 3:
   - 1 I Franzbranntwein
   - 20 g Kampfer
   - max. 250 mg Naproxen
   - 1 g Lidocain
   - 0,2 bis 0,5 g Prilocain
Zusammensetzung 4:
   - 1 I Franzbranntwein
   - 20 g Kampfer
   - 30 bis 90 mg Acemetacin
   - 1 g Lidocain
   - 0,2 bis 0,5 g Prilocain
Zusammensetzung 5:
   - 1 I Franzbranntwein
   - 20 g Kampfer
   - max. 8 mg Indometacin
   - 1 g Lidocain
   - 0,2 bis 0,5 g Prilocain
Zusammensetzung 6:
   - 1 I Franzbranntwein
   - 20 g Kampfer
   - 7,5 bis 15 mg Meloxicam
   - 1 g Lidocain
   - 0,2 bis 0,5 g Prilocain

## Patentansprüche

1. Pharmazeutische Zusammensetzung zur äußeren Anwendung, insbesondere zur Behandlung von Schmerzen und/oder Entzündungen, wobei die Zusammensetzung Franzbranntwein und Wirkstoffe umfasst, **dadurch gekennzeichnet, dass** die Wirkstoffe zumindest folgende weitere Bestandteile umfassen:
- Kampfer
- Lidocain
- Prilocain und/oder Procain
- eine Schmerzmittelkomponente.

2. Pharmazeutische Zusammensetzung nach dem vorhergehenden Anspruch, **dadurch gekennzeichnet, dass** der Anteil Kampfer, bezogen auf 1 I Franzbranntwein, ca. 15 bis 25 g, vorzugsweise 20 g, beträgt.

3. Pharmazeutische Zusammensetzung nach einem der vorhergehenden Ansprüche, **dadurch gekennzeichnet, dass** der Anteil Lidocain, bezogen auf 1 I Franzbranntwein, ca. 0,5 bis 1,5 g, vorzugsweise 1,0 g, beträgt.

4. Pharmazeutische Zusammensetzung nach einem der vorhergehenden Ansprüche, **dadurch gekennzeichnet, dass** der Anteil Prilocain und/oder Procain, bezogen auf 1 I Franzbranntwein, ca. 0,1 bis 1,0 g, vorzugsweise 0,2 bis 0,5 g, beträgt.

5. Pharmazeutische Zusammensetzung nach einem der vorhergehenden Ansprüche, **dadurch gekennzeichnet, dass** der Anteil der Schmerzmittelkomponente, bezogen auf 1 I Franzbranntwein, ca. 1,5 bis 2,5 g, vorzugsweise 2,0 g, beträgt.

6. Pharmazeutische Zusammensetzung nach dem vorhergehenden Anspruch, **dadurch gekennzeichnet, dass** die Schmerzmittelkomponente Diclofenac umfasst.

7. Pharmazeutische Zusammensetzung nach einem der vorhergehenden Ansprüche, **dadurch gekennzeichnet, dass** die Schmerzmittelkomponente Ibuprofen umfasst.

8. Pharmazeutische Zusammensetzung nach einem der vorhergehenden Ansprüche, **dadurch gekennzeichnet, dass** die Schmerzmittelkomponente Naproxen umfasst.

9. Pharmazeutische Zusammensetzung nach einem der vorhergehenden Ansprüche, **dadurch gekennzeichnet, dass** die Schmerzmittelkomponente Acemetacin umfasst.

10. Pharmazeutische Zusammensetzung nach einem der vorhergehenden Ansprüche, **dadurch gekennzeichnet, dass** die Schmerzmittelkomponente Indometacin umfasst.

11. Pharmazeutische Zusammensetzung nach einem der vorhergehenden Ansprüche, **dadurch gekennzeichnet, dass** die Schmerzmittelkomponente Meloxicam umfasst.

12. Pharmazeutische Zusammensetzung nach einem der vorhergehenden Ansprüche, **dadurch gekennzeichnet, dass** der Franzbranntwein ca. 60 bis 80 Vol.-% Alkohol, insbesondere ca. 70 Vol.-% Alkohol, enthält.

13. Pharmazeutische Zusammensetzung nach einem der vorhergehenden Ansprüche, **dadurch gekennzeichnet, dass** die Zusammensetzung flüssig ist.

14. Pharmazeutische Zusammensetzung nach einem der Ansprüche 1 bis 12, **dadurch gekennzeichnet, dass** die Zusammensetzung gelförmig ist.

15. Pharmazeutische Zusammensetzung nach dem vorhergehenden Anspruch, **dadurch gekennzeichnet, dass** die Zusammensetzung Methlyhydroxypropylcellulose enthält.

16. Sprühdose, vorzugsweise Pumpsprühdose oder treibgasbetriebene Sprühdose, **dadurch gekennzeichnet, dass** die Sprühdose mit einer pharmazeutischen Zusammensetzung nach einem der Ansprüche 1 bis 13 befüllt ist.
